## Europäisches Patentamt

## European Patent Office

## Office européen des brevets

(19)

(11) Veröffentlichungsnummer : **0 021 184**
**B1**

(12)

# EUROPÄISCHE PATENTSCHRIFT

(45) Veröffentlichungstag der Patentschrift :
08.02.84

(51) Int. Cl.³ : **A 61 K  9/00**, A 61 K  9/22,
A 61 K 37/24, C 07 C103/84

(21) Anmeldenummer : 80103150.1

(22) Anmeldetag : 06.06.80

(54) Sekretinpräparate mit verstärkter und protrahierter Wirkung, Verfahren zu Ihrer Herstellung sowie Dihydroxyben-zoyl-L-tyrosin.

(30) Priorität : 13.06.79 DE 2923878
03.04.80 DE 3013105

(43) Veröffentlichungstag der Anmeldung :
07.01.81 Patentblatt 81/01

(45) Bekanntmachung des Hinweises auf die Patenterteilung : 08.02.84 Patentblatt 84/06

(84) Benannte Vertragsstaaten :
AT BE CH DE FR GB IT LI NL

(56) Entgegenhaltungen :
DE-C-  929 664
FR-A- 2 123 547
NL-C-  109 489
RESEARCH DISCLOSURE, Nr. 169, Mal 1978, Selten
4-6, disclosure 16909, Havant, Hants, GB, "Stabiles
Sekretinpräparat"

(73) Patentinhaber : HOECHST AKTIENGESELLSCHAFT
Postfach 80 03 20
D-6230 Frankfurt am Main 80 (DE)

(72) Erfinder : Bickel, Martin, Dr.
Beethovenstrasse 40
D-6000 Frankfurt am Main (DE)
Erfinder : Geiger, Rolf, Prof. Dr.
Heinrich-Bleicher-Strasse 33
D-6000 Frankfurt am Main 50 (DE)
Erfinder : Leeb, Richard, Dr.
Nonnbornstrasse 15
D-6233 Kelkheim (Taunus) (DE)
Erfinder : Petri, Walter, Dr.
Panoramastrasse 19
D-6272 Niedernhausen/Taunus (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

Jouve, 18, rue St-Denis, 75001 Paris, France

EP 0 021 184 B1

Sekretinpräparate mit verstärkter und protrahierter Wirkung, Verfahren zu ihrer Herstellung sowie
Dihydroxybenzoyl-L-tyrosin

Die Erfindung betrifft Sekretinpräparate mit verstärkter und protrahierter Wirkung, insbesondere subcutan und reactal verabreichbare Sekretinpräparate.

Es wurde schon beschrieben, daß die Stabilität von Sekretin durch Salzbildung mit nicht-flüchtigen Säuren erhöht werden kann (2244 Research Disclosure No. 16909 (1978, 05)). Darüber hinaus hat man immer wieder versucht, die kurze Wirkung des Hormons Sekretin durch geeignete Injektionszubereitungen zu verlängern. In der NL-C 109 498 werden schwer lösliche bzw. unlösliche Komplexe von Peptidhormonen, die einen Depoteffekt zeigen, beschrieben. Insbesondere für Injektionszwecke sind jedoch leicht lösliche Zubereitungen erwünscht. Eine Präparation mit verlängerter Wirkung, nämlich eine Komposition aus Sekretin, Polyphloretinphosphat (PPP) und Gelatinederivaten wird in der DE-C 21 04 344 beschrieben.

Die Wirkung des PPP wird seiner hyaluronidasehemmenden Eigenschaft und seiner Fähigkeit, das basische Sekretin an sich zu binden, zugeschrieben. Die Wirkung des Gelatinederivats wird in einer zusätzlichen Verzögerung der Resorption des Sekretins gesehen, wobei auch die Adduktbildung beobachtet wurde. Bei diesen Präparaten besteht die Gefahr, daß es durch Ablagerung von PPP im Gewebe zu unerwünschten Reaktionen kommen kann. PPP ist außerdem wegen des undefinierten hohen Molekulargewichtes und wegen der undefinierten Struktur (Quervernetzung) für pharmazeutische Anwendundung unter Umständen wenig geeignet. Außerdem ist diese Substanz aufgrund der Anwesenheit von Pyridin aus toxikologischer Sicht nicht unproblematisch.

Viele Arzneimittel können in Suppositorien eingearbeitet und rectal verabreicht werden. Sie werden in dieser Form hinreichend gut resorbiert. Für Peptide sind Suppositorien bzw. Rectalkapseln keine geeignete Applikationsform, da hier für die Praxis die Resorptionsrate zu gering ist. Verabreicht man z. B. Sekretin auf diesem Wege, so beträgt die biologische Wirkung, die über die Pankreassaft-Sekretion gemessen wird, nur etwa 1 % von der bei subcutaner Injektion erzielten.

Es wurde nun überraschend gefunden, daß Dihydroxy-benzoyl-L-tyrosine, die keine Hyaluronidasehemmung zeigen, eine definierte Struktur besitzen und niedermolekular (im Vergleich zu PPP) sind, die Sekretinwirkung von Sekretininjektionspräparaten *in vivo* verstärken und verlängern.

Es wurde ferner gefunden, daß man die verfügbare Menge von Sekretin bei rectaler Applikation in Suppositorien beträchtlich steigern kann, wenn man der Suppositorienmasse die obengenannten Verbindungen zusetzt. Gleichzeitig verlängern sie die Wirkung des Sekretins, wie dies für Sekretininjektionspräparate ebenfalls beobachtet wurde. Die Erfindung betrifft daher ein Sekretinpräparat mit verstärkter und protrahierter Wirkung, das dadurch gekennzeichnet ist, daß es ein Dihydroxy-benzoyl-L-tyrosin als Depotkörper enthält.

In den vorstehenden und folgenden Ausführungen wird unter Sekretin natürliches und synthetisches Sekretin, insbesondere in Form von physiologisch verträglichen Salzen z. B. Hydrochlorid, Acetat oder Citrat, verstanden. Die Erfindung betrifft insbesondere Sekretininjektionspräparate sowie rectal verabreichbare Sekretinpräparate.

Durch die Anwendung der wie oben definierten Depotkörper vermeidet man die Gefahr einer Ablagerung von höhermolekularen Verbindungen im Gewebe, die zu unerwünschten Reaktionen führen kann. Eine deutliche Wirkungsverlängerung ist stets an das Vorhandensein mindestens einer freien phenolischen Gruppe im Molekül gebunden. Die Wirkungen gehen bei subcutaner und rectaler Applikation nicht immer parallel. So erzielt man z. B. mit einer typischen Verbindung, dem 3,5-Dihydroxy-benzoyl-L-tyrosin, rectal eine höhere Wirkungssteigerung als bei subcutaner Applikation.

Eine weitere Verzögerung der Sekretinwirkung wird bei den subcutan verabreichbaren Präparaten dadurch erreicht, daß das Präparat außer einem Dihydroxy-benzoyl-L-tyrosin noch ein Gelatinederivat enthält, das hergestellt wird entweder

1) durch Umsetzung von Kollagen-Abbauprodukten von einem Molekulargewicht von 2 000-20 000 mit einer geringen Menge Diisocyanat, als der Anzahl der im abgebauten Kollagen vorhandenen Amino- und Guanidino-Gruppen entspricht, bei 0-100 °C im neutralen oder schwach alkalischen Bereich und anschließende Einstellung des Vernetzungsproduktes au pH 7 und Isotonie mittels Natriumchlorid oder

2) durch weiteren Abbau des nach 1) erhaltenen Vernetzungsproduktes in wäßriger Lösung bei 60 bis 150 °C bis zu einem Molekulargewicht von 10 000 bis 100 000 und anschließende Einstellung auf pH 7 und Isotonie gemäß 1).

Sekretinpräparate zur subcutanen Applikation, die die in der nachfolgenden Tabelle genannten Depotkörper enthielten, wurden im Tiermodell geprüft. In der gewählten Versuchsanordnung erhielten je 5 bis 8 300 bis 500 g schwere männliche Ratten subkutan je 5 KE/kg Sekretin (KE bedeutet klinische Einheit) und 10 mg des zu prüfenden Depotkörpers in wäßriger Lösung der Suspension. Als Lösungsvermittler wurden N'-Methylpyrrolidon, Dimethylsulfoxid, Dimethylformamid, Tetrahydrofurfurylalkoholpolyäthylenglykoläther, Polyäthylenglykol, 1,3-Butylenglykol oder 1,2-Propylenglykol verwendet, welche für sich keine Verstärkung der Sekretinwirkung ergeben.

Die Tiere bleiben 18 Stunden vor dem Versuch nüchtern, erhalten Wasser ad libitum. In Urethannarkose (5 ml/kg i. m. Urethan 25 %) wird nach Tracheotomie das Abdomen mit Medianschnitt eröffnet und der Pylorus ligiert. Der Ductus choledochus wird in seinem proximalen Teil nahe der

# 0 021 184

Leberpforte unterbunden und die Galle über einen Polyäthylenschlauch, d = 0,05 mm, in das Duodenum geleitet. Der distale Teil des Gallenganges, in den die zahlreichen Pankreasgänge münden, wird unmittelbar vor seinem Eintritt in das Duodenum mittels eines Polyäthylenkatheters, d = 0,05 mm, kanüliert und das ablaufende Sekretvolumen in 60-minütigen Intervallen gemessen. Nach einem 60-minütigem Vorlauf werden die Präparate appliziert.

In der nachfolgenden Tabelle ist in Spalte 1 der Depotkörper aufgeführt. Die zweite Spalte sagt aus, um wieviel Prozente die Pankreassekretion nach 5 Stunden über der Basalsekretion liegt (bezogen auf Kontrolle = 100 %). Dieser Wert ist ein Maß für den Depoteffekt des geprüften Depotkörpers. Spalte 3 gibt die Wirkungsverstärkung von Sekretin an. Hier ist die gesamte Pankreassaftsekretion über 5 Stunden im Vergleich zur Kontrolle (0,9 % NaCl) = ) angegeben.

Tabelle

Pankreassaftsekretion der Ratte nach sc. Gabe von 5 KE/kg Sekretin
mit Dihydroxy-benzoyl-L-tyrosin (10 mg/Tier)

| 1<br>Depotkörper | 2<br>Δ % vs.<br>Kontrolle | 3<br>rel. Wirkungsstärke<br>über 5 Stunden |
|---|---|---|
| 3,5-Dihydroxy-benzoyl-L-tyrosin | 76 | 9,2 |
| 2,4-Dihydroxy-benzoyl-L-tyrosin | 84 | 10,5 |

Wie aus den Tabellen erkennbar, üben die untersuchten Depotkörper eine protrahierende und verstärkende Wirkung des Sekretins aus.

Daß diese Depotkörper nicht nur in Mischung mit Sekretin vorliegen, sondern auch mit letzterem Addukte bilden, erkennt man leicht am drastischen Rückgang der molekularen Extinktion der Dihydroxy-benzoyl-L-tyrosine im Bereich von 280 nm um bis zu 50 % unter Zugabe steigender Mengen Sekretin.

Da diese Adduktbildung jedoch reversibel ist und vom Massenwirkungsgesetz beherrscht wird, ist es vorteilhaft, einen großen molaren Überschuß an Depotkörper zu verwenden. Auf 80 bis 1 000 klinische Einheiten (KE ; 1 mg Sekretin = 4 000 KE ; nach Gut *19* (1978), Seite 355, soll 1 mg Sekretin eine biologische Wirkung entsprechend 5 000 KE ausüben) setzt man bei der subcutanen Anwendung am Menschen 10 bis 100 mg Dihydroxybenzoyl-L-tyrosin in 1 bis 2 ml Lösung oder Suspension ein. Gibt man zur Verbesserung der Wirkung ein hiervor definiertes Gelatinederivat zu, so beträgt dessen Konzentration 30 bis 100 mg/ml. Auch die in dem Sekretin gegebenenfalls vorhandenen Gelatinederivate erniedrigen die UV-Extinktion der Dihydroxy-benzoyl-L-tyrosine und liegen damit in komplexer Bindung vor. Zur Herstellung der Addukte und Mischungen genügt es, die Komponenten in Lösung kurze Zeit in Kontakt zu bringen. Die Adduktbildung erfolgt rasch.

Dihydroxy-benzoyl-L-tyrosin eignet sich als Depotkörper ebenfalls für rectal verabreichbaren Sekretinzubereitungen. Auch für diese Art der Zubereitungen ist es ein großer Vorzug, daß schon die einfach gebauten, niedermolekularen leicht zugänglichen Dihydroxy-benzoyl-L-tyrosine geeignet sind, die verfügbare Menge von Sekretin aus Zäpfchen oder Rectalkapseln so zu erhöhen, daß dadurch erstmals eine rectale Anwendung von Sekretin möglich bzw. sinnvoll ist. Es findet ebenfalls die erwähnte reversible Adduktbildung statt. Es ist in gleicher Weise vorteilhaft, einen großen molaren Überschuß an Depotkörper zu verwenden.

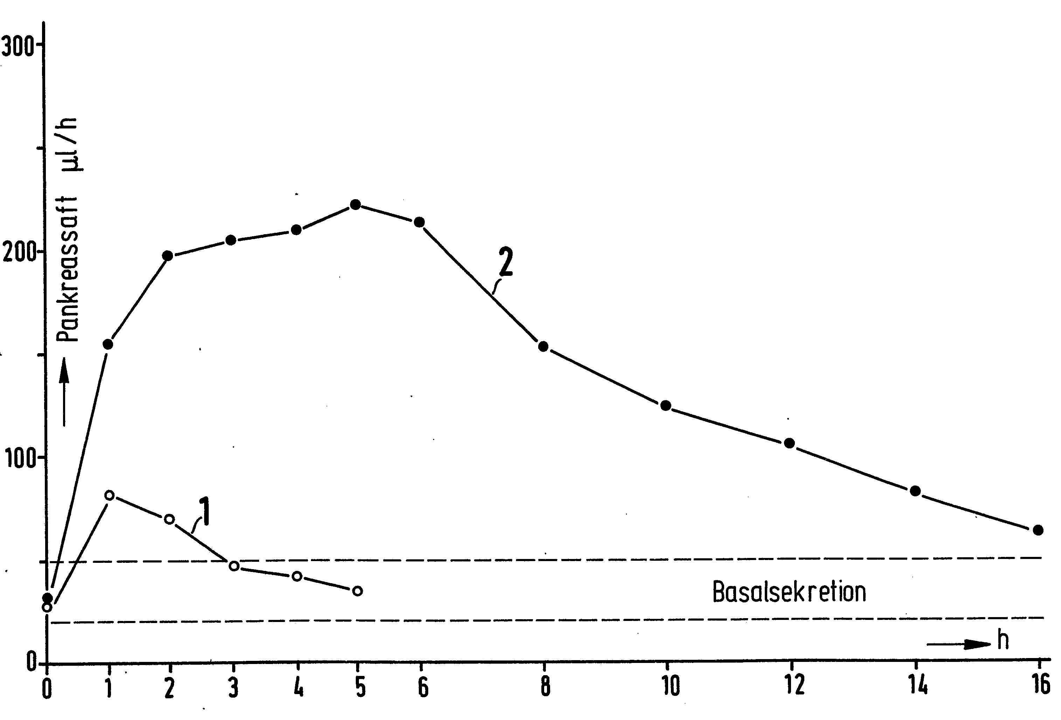

Die Wirkungsverstärkung von Sekretin in Form rectal verabreichbarer Präparate durch die erfindungsgemäß verwendeten Depotkörper wird aus der Abbildung deutlich. In einem Versuch an je 8 Ratten im Gewicht von 0,5 kg bewirken 100 µg Sekretin, in einem Suppositorium von 73 mg Gewicht die Produktion von 92 µl Pankreassaft (Kurve 1). Mit 25 mg 3,5-Dihydroxybenzoyl-L-tyrosin als Zusatz stieg das Pankreassaft-Volumen auf 1 778 µl (Kurve 2). Das entspricht einer etwa 20-fachen Wirkungssteigerung, Außerdem ist eine protrahierte Wirkung zu beobachten. Im vorliegenden Fall war die Sekretindosis sehr hoch gewählt, um auch ohne Zusatz ein meßbares Volumen zu erhalten.

Bei nichtmaximaler Stimulierung im normalen Dosisbereich (etwa halbe Dosierung) beträgt die Wirkungssteigerung das etwa 50-fache).

Für die Behandlung des Menschen benötigt man pro Zäpfchen etwa 0.1 bis 2 mg Sekretin ( = 400 bis 8 000 klinische Einheiten ; KE ; 1 mg Sekretin = 4 000 KE ; nach Gut 19 (1978), Seite 355 soll 1 mg Sekretin eine biologische Wirkung entsprechend 5 000 KE ausüben). Die Mindestmenge an Depotkörper liegt bei etwa 50 mg. Die Höchstmenge ist von der Verarbeitbarkeit bestimmt. Sie beträgt bei einem Zäpfchen von etwa 2 g Gewicht bis zu 1-1,5 g.

Gegenstand der Erfindung ist auch ein Verfahren zur Herstellung eines Sektretinpräparates mit protrahierter und verstärkter Wirkung.

Bei der Herstellung eines erfindungsgemäßen subcutan verabreichbaren Präparates geht man

3

vorteilhaft so vor, daß man Sektretinhydrochlorid, hergestellt nach US-A-4 098 779 (entsprechend DE-A-2 615 229) in Wasser oder in wäßriger Lösung eines wie oben definierten Gelatinederivates löst und mit der Lösung eines Dihydroxy-benzoyl-L-tyrosins in Wasser vereinigt. Das pH liegt bei diesem Vorgang zwischen 7 und 8,5. Man stellt mit einer physiologisch verträglichen Säure auf pH 7,0 bis 7,8 ein und lyophilisiert anschließend.

Weiterhin ist es möglich, die Depotkörper auch in Wasser, welches bis zu 30 % eines Lösungsvermittlers enthält, zu lösen. Als Lösungsvermittler kommen insbesondere in Betracht : 1,2-Propylenglykol, 1,3-Butylenglykol, Polyäthylenglykol, Tetrahydrofurfurylalkohol-polyethylengklykolether, Dimethylsulfoxid, N-Methylpyrrolidon oder Dimethylformamid. Man stellt in diesem Fall vorzugsweise die Lösung des Depotkörpers ohne Sekretin her und füllt diese Lösung, welche nicht lyophilisiert wird, in Ampullen zu vorzugsweise 1-2 ml ab. In einer zweiten Ampulle befindet sich das Sekretin in gefriergetrocknetem Zustand. Unmittelbar vor Applikation wird das Sekretin in der Lösung des Depotkörpers gelöst, wobei dann das Addukt bildet. Ein gegebenenfalls zugesetztes Gelatinederivat kann sowohl dem Sekretin als auch dem Depotkörper oder beiden zugegeben werden.

Das Verfahren zur Herstellung von rectal verabreichbaren Sekretinpräparaten mit protrahierter und verstärkter Wirkung, ist dadurch gekennzeichnet, daß man aus einem Gemisch aus einem allgemein üblichen Trägerstoff, Sekretin und einem Dihydroxybenzoyl-L-tyrosin als Depotkörper Suppositorien oder Rectalkapseln herstellt.

Als Suppositorienmasse verwendet man vorbekannte Verbindungen, zum Beispiel Partialglyceride, d.h. Mischungen aus Mono- und Diestern des Glycerins mit höheren Fettsäuren, ferner Fettsäure-1,2-propylenglykolester, ferner Polyethylenglykole mit einem Erstarrungsbereich zwischen etwa 30 und 50 °C. Die in solche Massen oder in Öl zusammen mit einer Dihydroxy-benzoyl-L-tyrosin eingearbeitete Sekretinsuspension kann auch in handelsübliche Rectalkapseln abgefüllt werden. Zur Herstellung der Suppositorien geht man so vor, daß das Gemisch aus Trägerstoff, Sekretin und Dihydroxybenzoyl-L-tyrosin in Schmelze homogenisiert und anschließend noch flüssig in Suppositorienformen abgefüllt wird, in denen man es erstarren läßt. Halbfeste oder ölige Suspensionen werden in Rectalkapseln abgefüllt. Das Gewicht eines Suppositoriums beträgt etwa 1-3 g.

In ähnlicher Weise kann man auch Suppositorien auf Glycerin-Gelatine-Grundlage herstellen.

Suppositorienmassen wie Fettsäure 1,2-propylenglykolester werden zusammen mit Sekretin und einem erfindungsgemäß verwendbaren Depotkörper gemischt und ohne Schmelzen als Granulat verpreßt. Die erfindungsgemäßen Suppositorien bzw. Rectalkapseln werden zur Behandlung und Verhütung von Hämmorhagien des Intestinaltraktes und zur Behandlung von Ulcera verwendet. Sie stellen die erste Sekretinzubereitung dar, die nicht mehr durch Injektion verabreicht werden muß.

Die Erfindung betrifft ferner Dihydroxybenzoyl-L-tyrosin, das als Depotkörper für die erfindungsgemäßen Sekretinzubereitungen verwendet wird. Insbesondere betrifft sie das 3,5-Dihydroxybenzoyl-L-tyrosin. Die Herstellung ist dadurch gekennzeichnet, daß man Dihydroxybenzoesäure mit L-Tyrosinmethylester umsetzt und den erhaltenen Ester verseift.

Die phenolischen OH-Gruppen können zum Beispiel durch Acetylierung während der Amidsynthese geschützt sein. Sie werden vom Reaktionsprodukt in an sich bekannter Weise durch Behandeln mit Alkali, Ammoniak oder Aminen abgespalten. Es ist jedoch auch möglich, die Amidbindung ohne vorherigen Schutz der OH-Gruppen herzustellen, wenn man die Kondensation mittels Cardodiimid in Anwesenheit eines Zusatzes wie 1-Hydroxybenzotriazol vornimmt (Chem. Ber. 103 (1970) Seite 788-798).

Die erfindungsgemäßen Depotkörper können partiell als Salze z. B. Alkali-oder Erdalki-salze oder als Salze mit organischen Basen wie z. B. Tris-hydroxymethylaminomethan vorliegen. Die Salze erhält man, indem man z. B. die entsprechende Verbindung in Wasser löst oder suspendiert und unter Rühren solange Base hinzugibt, bis ein pH von 7,0-7,5 erreicht und gehalten wird.

Die erfindungsgemäßen Sekretinzubereitungen werden zur Behandlung und Verhütung von Hämmorhagien des Intestinaltrakts und zur Behandlung von Ulcera verwendet. Die Präparate werden dabei durch Injektion oder rectal verabreicht.

In den nachfolgenden Beispielen sind allgemeine, leicht auszuführende Vorschriften zur Herstellung von Dihydroxybenzoyl-L-tyrosin und rectal bzw. subcutan verabreichbaren Depotzubereitungen angegeben.

## Beispiel 1

3,5-Dihydroxybenzoyl-L-tyrosin

a) 3,5-Dihydroxybenzoyl-L-tyrosin-methylester

231,2 g 3,5-Dihydroxybenzoesäure werden in 4 l Dimethylformamid gelöst. Man gibt 313 g L-Tyrosinmethylesterhydrochlorid, 202 g 1-Hydroxybenzotriazol und 346 ml N-Ethylmorpholin und anschließend 297 g Dicyclohexylcarbodiimid zu. Dann rührt man über Nacht bei Raumtemperatur und filtriert den ausgefallenen Dicyclohexylharnstoff ab. Das Lösungsmittel wird im Vakuum abdestilliert, der Rückstand in 4 l Essigester aufgenommen, die Essigesterlösung je 4 mal mit gesättigter Natriumhydrogencarbonatlösung, 3 mal mit 400 ml einer Lösung aus 5 % $KHSO_4$ und 10 % $K_2SO_4$ und zweimal mit 200 ml Wasser gewaschen, über Natriumsulfat getrocknet und anschließend zur Trockne gebracht. Ausb. 360 g Harz.

b) 3,5-Dihydroxybenzoyl-L-tyrosin

Man löst das wie oben erhaltene Harz in etwa 1,4 l 2N NaOH und bringt mit 6N NaOH auf pH 12,5. Unter Stickstoff und pH-Kontrolle wird durch Zugabe von 2N NaOH bei konstantem pH 12.5 verseift, Dauer ca. 15 Stunden. Die alkalische Lösung wird 4 mal mit je 500 ml n-Butanol extrahiert. Die wäßrige Phase wird mit 6 N HCl neutralisiert, 10 Minuten mit Aktivkohle (50 g) behandelt, filtriert und weiter angesäuert, solange sich ein Öl absetzt. Dieses Öl wird 1 l n-Butanol aufgenommen, die wäßrige Phase noch zweimal mit je 300 ml n-Butanol extrahiert. Die vereinigten Butanolphasen werden zweimal mit je 100 ml gesättigter Kochsalzlösung und einmal mit 150 ml Wasser gewaschen, über Natriumsulfat getrocknet, filtriert und im Vakuum vom Lösungsmittel befreit. Das zurückbleibende Öl wird in 600 ml Aceton gelöst. Man filtriert und tropft die Lösung unter kräftigem Rühren in 8 l Chloroform. Der Niederschlag wird gesammelt, mit Chloroform gewaschen und im Vakuum getrocknet. Ausbeute 255 g eines schwach hygroskopischen Pulvers. Die Verbindungen und ihr Dicyclohexylaminsalz schmelzen unter Zersetzung.

$C_{16}H_{15}NO_6$, 0,5 $H_2O$ ; 326,3 ;

Ber.   C 58,89   H 4,94   N 4,29

Gef.   C 58,9    H 5,2    N 4,2

In analoger Weise ist 2,4-Dihydroxybenzoyl-L-tyrosin erhältlich.

## Beispiel 2

Man homogenisiert bei 40-60 °C eine Suspension aus 0,5 bis 1 g Sekretinhydrochlorid und 250 g 3,5-Dihydroxybenzoyl-L-tyrosin in 480 g Suppositorienmasse, bestehend aus einem Partialglycerid oder Polyethylenglykol mit einem Erstarrungspunkt von jeweil 30 bis 50 °C, und füllt noch flüssig bzw. halbflüssig in Suppositorienformen ab. Das Gewicht eines Suppositoriums is etwa 2 g.

## Beispiel 3

Man arbeitet wie unter Beispiel 2 beschrieben, setzt jedoch nur 0,05 g Sekretin-hydrochlorid ein.

## Beispiel 4

Man arbeitet nach Beispiel 2 oder 3, setzt 250 g des Natriumsalzes von 3,5-Dihydroxybenzoyl-L-tyrosin ein.

## Beispiel 5

Man arbeitet nach Beispiel 2, 3 oder 4, verwendet jedoch 1 g Sekretin-Citrat und 1 000 g Suppositorienmasse.

## Beispiel 6

Man homogenisiert eine Mischung von 0,5 g Sekretin-Citrat, 200 g 3,5-Dihydroxybenzoyl-L-tyrosin oder einen seiner physiologisch verträglichen Salze und 500 g eines partiell hydrierten Pflanzenöls, z. B. Sesam-, Sojabohnen-, Palm- oder Rüböl und füllt je 2,0 g in Rectalkapseln ab.

## Beispiel 7

Man arbeitet 1 g Sekretin-hydrochlorid in eine Mischung von 250 g 3,5-Dihydroxybenzoyl-L-tyrosinnatriumsalz und 500 g Fettsäure-1,2-propylenglykolester ein, granuliert und preßt in Suppositorienformen ein.

## Beispiel 8

Man löst bei etwa 50 °C unter Rühren 0,5 g Sekretin-hydrochlorid in einer Lösung von 110 g Gelatine und 90 g Glycerin in 300 ml Wasser und vermischt mit 200 g Tris-hydroxymethyl-aminomethansalz von 3,5-Dihydroxybenzoyl-L-tyrosin und füllt die Mischung in Suppositorienformen und kühlt rasch ab.

## Beispiel 9

Man arbeitet nach Beispiel 2 bis 8, verwendet jedoch 2,4-Dihydroxybenzoyl-L-tyrosin in entsprechender Gewichtsmenge.

## Beispiel 10

Man löst zum Beispiel

5

A. 30 g 2,4-Dihydroxybenzoyl-L-tyrosin in 300 ml Wasser
B. 80 g Gelatinederivat in 500 ml Wasser
C. 1 Million KE Sekretin und 2 g NaCl in 100 ml Wasser.

Die Lösungen A-C werden gereinigt und mit Wasser auf 1,0 Liter aufgefüllt. Man filtriert steril, füllt unter aseptischen Bedingungen in Ampullen zu 1 ml ab und lyophilisiert.

## Beispiel 11

Man verfährt nach Beispiel 10 vereinigt jedoch nur die beiden Lösungen A und B, welche auf 1 l aufgefüllt, in Ampullen zu 1 ml abgefüllt und lyophilisiert werden. Lösung C enthält neben 1 Million KE Sektretin 20 g Glycin und 2 g Gelatinederivat und wird getrennt in derselben Weise in Ampullen zu je 1 000 KE abgefüllt und lyophilisiert. Vor Verwendung wird der Depotkörper in 1 ml Wasser gelöst. In dieser Lösung nimmt man das lyophilisierte Sekretin auf und injiziert.

## Beispiel 12

Man löst 30 g 2,4-Dihydroxybenzoyl-L-tyrosin in einer Mischung aus 200 ml 1,2-Propylenglykol und 200 ml Wasser und vereinigt diese Lösung mit der Lösung von 80 g Gelatinederivat in 500 ml Wasser. Es wird mit Wasser auf 1 l aufgefüllt, steril filtriert und in Ampullen zu je 1 ml abgefüllt. Diese Lösung wird dazu verwendet, das nach Beispiel 11 hergestellte Sekretin aufzunehmen. Nach Auflösen des Ampulleninhalts kann sofort injiziert werden.

## Beispiel 13

Man löst 30 g 2,4-Dihydroxybenzoyl-tyrosin analog Beispiel 12 in 200 ml Propylenglykol und 800 ml Wasser, filtriert steril und füllt in Ampullen zu je 1 ml ab. Zur Verwendung wird das nach Beispiel 11 ampullierte Sekretin in dieser Lösung aufgenommen und injiziert.

## Beispiel 14

Man löst 30 g 2,4-Dihydroxybenzoyl-L-tyrosin in 800 ml Wasser unter Zugabe von 1N NaOH bis etwa pH 8,5. Dann wird mit 1N HCl auf pH 7,4 eingestellt, mit Wasser auf 1,0 l aufgefüllt, filtriert, in Ampullen zu je 1 ml abgefüllt und lyophilisiert. Zur Verwendung wird der Ampulleninhalt in 1 ml Wasser aufgenommen und darin das nach Beispiel 11 ampullierte Sekretin gelöst.

## Beispiel 15

Man verfährt nach Beispiel 10 bis 12 setzt jedoch nur 15 g 2,4-Dihydroxybenzoyl-L-tyrosin und gegebenenfalls 40 g eines Gelatinederivats ein.

## Beispiel 16

Man verfährt nach Beispiel 10 bis 12 setzt jedoch 60 g 2,4-Dihydroxybenzoyl-L-tyrosin und gegebenenfalls 80 g eines Gelatinederivats ein.

## Beispiel 17

Man verwendet 3,5-Dihydroxybenzoyl-L-tyrosin, wobei die Verbindung je nach Löslichkeit im Sinne der Beispiele 10 bis 14 eingesetzt wird.

**Ansprüche** (für die Vertragsstaaten : BE, CH, DE, FR, GB, IT, LI, NL)

1. Sekretinpräparat mit verstärkter und protrahierter Wirkung, dadurch gekennzeichnet, daß es ein Dihydroxybenzoyl-L-tyrosin als Depotkörper enthält.
2. Sekretinpräparat gemäß Anspruch 1, dadurch gekennzeichnet, daß es 3,5 oder 2,4-Dihydroxybenzoyl-L-tyrosin als Depotkörper enthält.
3. Subcutan verabreichbares Sekretinpräparat gemäß einem der Ansprüche 1 oder 2.
4. Sekretinpräparat gemäß Anspruch 3, dadurch gekennzeichnet, daß es zusätzlich ein Gelatinederivat enthält, das in bekannter Weise hergestellt wird entweder
   1) durch Umsetzung von Kollagen-Abbauprodukten von einem Molekulargewicht von 2 000 bis 20 000 mit einer geringeren Menge Diisocyanat als der Anzahl der im abgebauten Kollagen vorhandenen Amino- und Guanidino-Gruppen entspricht, bei 0 bis 100 °C im neutralen oder schwach alkalischen Bereich und anschließende Einstellung des Vernetzungsproduktes auf pH 7 und Isotonie mittels Natriumchlorid oder

2) durch weiteren Abbau des nach 1) erhaltenen Vernetzungsproduktes in wäßriger Lösung bei 60 bis 150 °C bis zu einem Molekulargewicht von 10 000 bis 100 000 und anschließende Einstellung auf pH 7 und Isotonie gemäß 1).

5. Verfahren zur Herstellung eines Sekretinpräparates mit protrahierter und verstärkter Wirkung gemäß Anspruch 3 dadurch gekennzeichnet, daß man eine wäßrige Lösung von Sekretin, mit einer wäßrigen, gegebenenfalls einen physiologisch verträglichen Lösungsmittler enthaltenden Lösung oder Suspension von Dihydroxybenzoyl-L-tyrosin vereinigt, wobei eine der Lösungen gegebenenfalls noch ein Gelatinederivat gemäß Anspruch 4 enthält.

6. Verfahren zur Herstellung eines Sekretinpräparates gemäß Anspruch 3, dadurch gekennzeichnet, daß man lyophilisiertes Sekretin oder die lyophilisierte Mischung von Sekretin mit einem Gelatinederivat gemäß Anspruch 4 in der wäßrigen, gegebenenfalls einen Lösungsvermittler und/oder ein Gelatinederivat gemäß Anspruch 4 enthaltenden Lösung oder Suspension von Dihydroxybenzoyl-L-tyrosin aufnimmt.

7. Rectal verabreichbares Sekretinpräparat gemäß Anspruch 1 oder 2.

8. Sekretinpräparat nach Anspruch 7, dadurch gekennzeichnet, daß es als Depotkörper 3,5-Dihydroxybenzoyl-L-tyrosin enthält.

9. Verfahren zur Herstellung eines rectal verabreichbaren Sekretinpräparates mit verstärkter und protrahierter Wirkung gemäß Anspruch 7, dadurch gekennzeichnet, daß man aus einem Gemisch aus allgemein üblichen Trägerstoffen, Sekretin und Dihydroxybenzoyl-L-tyrosin, Suppositorien oder Rectalkapseln herstellt.

10. Dihydroxy-benzoyl-L-tyrosin.


**Ansprüche** (für den Vertragsstaat AT)

1. Verfahren zur Herstellung eines Sekretinpräparates mit verstärkter und protrahierter Wirkung, dadurch gekennzeichnet, daß man Sekretin und Dihydroxybenzoyl-L-tyrosin als Depotkörper, mit einem pharmakologisch verträglichen Trägerstoff mischt, darin löst oder suspendiert und in eine Dosierungseinheit überführt.

2. Verfahren gemäß Anspruch 1, dadurch gekennzeichnet, daß man 3,5-oder 2,4-Dihydroxybenzoyl-L-tyrosin als Depotkörper einsetzt.

3. Verfahren gemäß Anspruch 1 oder 2 zur Herstellung eines subcutan verabreichbaren Sekretinpräparates mit protrahierter und verstärkter Wirkung dadurch gekennzeichnet, daß man eine wäßrige Lösung von Sekretin, mit einer wäßrigen, gegebenenfalls einen physiologisch verträglichen Lösungsvermittler enthaltenden Lösung oder Suspension von Dihydroxybenzoyl-L-tyrosin vereinigt, wobei eine der Lösungen gegebenenfalls noch ein Gelatine-derivat enthält, das in bekannter Weise hergestellt wird entweder.

1) durch Umsetzung von Kollagen-Abbauprodukten von einem Molekulargewicht von 2 000 bis 20 000 mit einer geringeren Menge Diisocyanat als der Anzahl der im abgebauten Kollagen vorhandenen Amino- und Guanidino-Gruppen entspricht, bei 0 bis 100 °C im neutralen oder schwach alkalischen Bereich und anschließende Einstellung des Vernetzungsproduktes auf pH 7 und Isotonie mittels Natriumchlorid oder

2) durch weiteren Abbau des nach 1) erhaltenen Vernetzungsproduktes in wäßriger Lösung bei 60 bis 150 °C bis zu einem Molekulargewicht von 10 000 bis 100 000 und anschließende Einstellung auf pH 7 und Isotonie gemäß 1.

4. Verfahren gemäß Anspruch 1 oder 2 zur Herstellung eines Sekretinpräparates, dadurch gekennzeichnet, daß man lyophilisiertes Sekretin oder die lyophilisierte Mischung von Sekretin mit einem Gelatinederivat gemäß Anspruch 3 in der wäßrigen, gegebenenfalls einen Lösungsvermittler und/oder ein Gelatinederivat gemäß Anspruch 3 enthaltenden Lösung oder Suspension von Dihydroxybenzoyl-L-tyrosin aufnimmt.

5. Verfahren gemäß Anspruch 1 oder 2 zur Herstellung eines rectal verabreichbaren Sekretinpräparates mit verstärkter und protrahierter Wirkung, dadurch gekennzeichnet, daß man aus einem Gemisch aus allgemein üblichen Trägerstoffen, Sekretin und Dihydroxybenzoyl-L-tyrosin, Suppositorien oder Rectalkapseln herstellt.


**Claims** (for the Contracting States : BE, CH, DE, FR, GB, IT, LI, NL)

1. Secretin preparation with an intensified and protracted action, which contains a dihydroxybenzoyl-L-tyrosine as depot body.

2. Secretin preparation according to claim 1, which contains 3.5- or 2.4-dihydroxy-benzoyl-L-tyrosine as depot body.

3. Subcutaneously administrable secretin preparation according to one of claims 1 or 2.

4. Secretin preparation as claimed in claim 3, which contains additionally a gelatin derivative which is prepared in known manner either.

1) by a reaction of collagen degradation products of a molecular weight of from 2,000 to 20,000 with an amount of diisocyanate smaller than that corresponding to the number of the amino and guanidino groups present in the degraded collagen, at 0 to 100 °C in the neutral or slightly alkaline range and subsequent adjustment of the cross-linked product to a pH value of 7 and to isotonicity by way of sodium chloride, or

2) by a further degradation of the cross-linked product obtained according to step 1) in aqueous solution at 60 to 150 °C up to a molecular weight of from 10,000 to 100,000 and subsequent adjustment to pH 7 and isotonicity according to 1) above.

5. Process for the manufacture of a secretin preparation with a protracted and intensified action as claimed in claim 3, which comprises combining an aqueous solution of secretin with an aqueous solution or suspension of dihydroxy-benzoyl-L-tyrosine, wherein the solution or suspension of dihydroxy-benzoyl-L-tyrosine may contain a physiologically acceptable solubilizer, and one of the two solutions may also contain a gelatin derivative according to claim 4.

6. Process for the manufacture of a secretin preparation as claimed in claim 3, which comprises dissolving lyophilized secretin or the lyophilized mixture of secretin with a gelatin derivative according to claim 4 in the aqueous solution or suspension of dihydroxybenzoyl-L-tyrosine, which solution or suspension may contain a solubilizer and/or a gelatin derivative according to claim 4.

7. Rectally administrable secretin preparation according to claim 1 or 2.

8. Secretin preparation as claimed in claim 7, which contains as depot body 3.5-dihydroxybenzoyl-L-tyrosine.

9. Process for the manufacture of a rectally administrable secretin preparation with an intensified and protracted action as claimed in claim 7, which comprises preparing suppositories or rectal capsules from a mixture of generally common carrier substances, secretin and dihydroxy-benzoyl-L-tyrosine.

10. Dihydroxy-benzoyl-L-tyrosine.


**Claims** (for the Contracting State AT)

1. Process for the manufacture of a secretin preparation with a intensified and protracted action wherein secretin and dihydroxy-benzoyl-L-tyrosine as depot body are mixed with or dissolved or suspended in a physiologically acceptable carrier substance and brought in a dosage unit.

2. Process according to claim 1 wherein 3.5- or 2.4-dihydroxyl-benzoyl-L-tyrosine is used as depot body.

3. Process according to claim 1 or 2 for the manufacture of a subcutaneously administrable secretin preparation with a protracted and intensified action which comprises combining an aqueous solution of secretin with an aqueous solution or suspension of dihydroxy-benzoyl-L-tyrosine wherein the solution or suspension of dihydroxybenzoyl-L-tyrosine may contain a physiologically acceptable solubilizer, and one of the two solutions may also contain a gelatine derivative, which is prepared in known manner either

1) by a reaction of collagen degradation products of a molecular weight of from 2,000 to 20,000 with an amount of diisocyanate smaller than that corresponding to the number of the amino and guanidino groups present in the degraded collagen, at 0 to 100 °C in the neutral or slightly alkaline range and subsequent adjustment of the cross-linked product to a pH value of 7 and to isotonicity by way of sodium chloride, or

2) by a further degradation of the cross-linked product obtained according to step 1) in aqueous solution at 60 to 150 °C up to a molecular weight of from 10,000 to 100,000 and subsequent adjustment to pH 7 and isotonicity according to 1) above.

4. Process according to claim 1 or 2 for the manufacture of a secretin preparation, which comprises dissolving lyophilized secretion or the lyophilized mixture of secretin with a gelatin derivative according to claim 3 in the aqueous solution or suspension of dihydroxy-benzoyl-L-tyrosine, which solution or suspension may contain a solubilizer and/or gelatin derivative according to claim 3.

5. Process according to claim 1 or 2 for the manufacture of a rectally administrable secretin preparation with an intensified and protracted action which comprises preparing suppositories or rectal capsules from a mixture of generally common carrier substances, secretin and dihydroxy-benzoyl-L-tyrosine.


**Revendications** (pour les Etats contractants : BE, CH, DE, FR, GB, IT, LI, NL)

1. Médicament à base de sécrétine à effet renforcé et prolongé, caractérisé en ce qu'il contient une dihydroxybenzoyl-L-tyrosine en tant que constituant à effet retard.

2. Médicament à base de secrétine selon la revendication 1, caractérisé en ce qu'il contient la 3,5- ou la 2,4-dihydroxybenzoyl-L-tyrosine en tant que constituant à effet retard.

3. Médicament à base de secrétine administrable par voie sous-cutanée selon la revendication 1 ou 2.

4. Médicament à base de secrétine selon la revendication 3, caractérisé en ce qu'il contient en outre un dérivé de gélatine, qui est préparé d'une manière connue, soit :

1) par réaction de produits de dégradation du collagène ayant une masse moléculaire de 2 000 à 20 000 avec une plus faible quantité de diisocyanate que le nombre correspondant de groupes amino et guanidino présents dans le collagène dégradé, entre 0 et 100 °C dans un domaine neutre ou faiblement alcalin, puis ajustage du produit de réticulation à un pH de 7 et réglage de l'isotonie avec du chlorure de sodium, soit :

2) par une autre dégradation du produit de réticulation obtenu en 1) en solution aqueuse entre 60 et 150 °C, jusqu'à une masse moléculaire de 10 000 à 100 000, puis ajustage à un pH de 7 et réglage de l'isotonie selon 1).

5. Procédé pour la préparation d'un médicament à base de secrétine à effet renforcé et prolongé selon la revendication 3, caractérisé en ce qu'on réunit une solution aqueuse de secrétine avec une solution ou une suspension aqueuse de dihydroxybenzoyl-L-tyrosine, contenant éventuellement un adjuvant de dissolution physiologiquement acceptable, une des solutions contenant encore éventuellement un dérivé de gélatine selon la revendication 4.

6. Procédé pour la préparation d'un médicament à base de secrétine selon la revendication 3, caractérisé en ce qu'on reprend la secrétine lyophilisée ou le mélange lyophilisé de secrétine avec un dérivé de gélatine selon la revendication 4, dans la solution ou la suspension aqueuse de dihydroxybenzoyl-L-tyrosine contenant éventuellement un adjuvant de dissolution et/ou un dérivé de gélatine selon la revendication 4.

7. Médicament à base de secrétine administrable par voie rectale selon la revendication 1 ou 2.

8. Médicament à base de secrétine selon la revendication 7, caractérisé en ce qu'il contient de la 3,5-dihydroxybenzoyl-L-tyrosine en tant que constituant à action retard.

9. Procédé pour la préparation d'un médicament à base de secrétine administrable par voie rectale, à effet renforcé et prolongé, selon la revendication 7, caractérisé en ce qu'on prépare des suppositoires ou des gélules rectales à partir d'un mélange de véhicules usuels, de secrétine et de dihydroxybenzoyl-L-tyrosine.

10. Dihydroxy-benzoyl-L-tyrosine.

**Revendications** (pour l'Etat contractant AT)

1. Procédé pour la préparation d'un médicament à base de secrétine à effet renforcé et prolongé, caractérisé en ce qu'on mélange la secrétine et la dihydroxybenzoyl-L-tyrosine en tant que constituant à effet retard, avec un véhicule pharmacologiquement acceptable, puis on dissout ou on met en suspension et on convertit en une dose unitaire.

2. Procédé selon la revendication 1, caractérisé en ce qu'on utilise la 3,5- ou la 2,4-dihydroxybenzoyl-L-tyrosine en tant que constituant à effet retard.

3. Procédé selon l'une des revendications 1 et 2 pour la préparation d'un médicament à base de secrétine, à effet prolongé et renforcé, administrable par voie sous-cutanée, caractérisé en ce qu'on réunit une solution aqueuse de secrétine à une solution ou une suspension aqueuse de dihydroxybenzoyl-L-tyrosine contenant éventuellement un adjuvant de dissolution physiologiquement acceptable, une des solutions contenant encore éventuellement un dérivé de gélatine qu'on prépare d'une manière connue, soit :

1) par réaction de produits de dégradation du collagène d'une masse moléculaire de 2 000 à 20 000 avec une plus faible quantité de diisocyanate que le nombre correspondant de groupes amino guanidino présents dans le collagène dégradé, entre 0 et 100 °C dans un domaine neutre ou faiblement alcalin, puis en ajustant le pH du produit de réticulation à une valeur de 7 et en réglant l'isotonie au moyen de chlorure de sodium, soit

2) par une autre dégradation du produit de réticulation obtenu en 1), en solution aqueuse entre 60 et 150 °C, jusqu'à une masse moléculaire de 10 000 à 100 000, puis ajustage à un pH de 7 et réglage de l'isotonie selon 1).

4. Procédé selon la revendication 1 ou 2, pour la préparation d'un médicament à base de secrétine, caractérisé en ce qu'on reprend la secrétine lyophilisée ou le mélange lyophilisé de secrétine avec un dérivé de gélatine selon la revendication 3, dans la solution ou la suspension aqueuse de dihydroxybenzoyl-L-tyrosine, contenant éventuellement un adjuvant de dissolution et/ou un dérivé de gélatine selon la revendication 3.

5. Procédé selon la revendication 1 ou 2, pour la préparation d'un médicament à base de secrétine à effet renforcé et prolongé, administrable par voie rectale, caractérisé en ce qu'on prépare des suppositoires ou des gélules rectales à partir d'un mélange de véhicules usuels, de secrétine et de dihydroxybenzoyl-L-tyrosine.